(19) Europäisches Patentamt
European Patent Office
Office européen des brevets

(11) Publication number: 0 037 710 B1

# (12) EUROPEAN PATENT SPECIFICATION

(45) Date of publication of patent specification: 01.08.84

(51) Int. Cl.³: C 07 D 277/30, A 61 K 31/425

(21) Application number: 81301428.9

(22) Date of filing: 02.04.81

## (54) Thiazole derivatives.

(30) Priority: 03.04.80 GB 8011251
26.04.80 GB 8013860

(43) Date of publication of application:
14.10.81 Bulletin 81/41

(45) Publication of the grant of the patent:
01.08.84 Bulletin 84/31

(84) Designated Contracting States:
AT DE NL SE

(56) References cited:
FR - A - 950 290
FR - A - 1 561 433
FR - A - 2 096 994
FR - A - 2 146 575
FR - M - 6 190
GB - A - 1 137 529
GB - A - 1 145 884
GB - A - 1 262 292
GB - A - 1 574 583
US - A - 3 539 585

The file contains technical information submitted after the application was filed and not included in this specification

(73) Proprietor: JOHN WYETH & BROTHER LIMITED
Huntercombe Lane South Taplow
Maidenhead Berkshire, SL6 0PH (GB)

(72) Inventor: Cavalla, John Frederick
105 The Grove
Isleworth Middlesex (GB)
Inventor: Franklin, Richard Arthur
17 Chaseside Avenue Twyford
Reading Berkshire (GB)

(74) Representative: Wileman, David Francis et al.
c/o John Wyeth and Brother Limited
Huntercombe Lane South
Taplow Maidenhead Berkshire SL6 0PH (GB)

(56) References cited:
JOURNAL OF MEDICINAL CHEMISTRY, vol. 17, no. 11, November 1974, K. BROWN et al. "Nonsteroidal antiinflammatory agents 1,2,4-diphenylthiazole-5-acetic acid and related compounds", pages 1177-1181

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 037 710 B1

(56) References cited:
CHEMICAL ABSTRACTS, vol. 93, no. 19, November 10, 1980 page 12, abstract 179303w Columbus, Ohio, USA S. FUMERO et al. "Metabolism of fentiazac"

CHEMICAL ABSTRACTS, vol. 89, no. 17, October 23, 1978 page 12, abstract 140156t Columbus, Ohio, USA PEDRAZZINI S. "Epicutaneous absorption of fentiazac"

## Description

This invention relates to topical pharmaceutical compositions containing thiazoles, to certain of the thiazoles, their preparation and the compounds for use as anti-inflammatory agents.

In our GB—A—1,145,884 there are described and claimed compounds of the general formula (A)

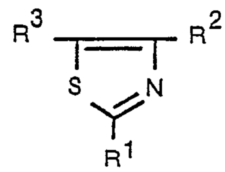

(A)

and acid addition salts thereof, in which $R_1$ and $R_2$ are the same or different and are substituted aryl radicals (which may be heteroaryl radicals) and $R^3$ is a lower aliphatic carboxylic acid radical containing from 2 to 6 carbon atoms or a salt, ester, amide, nitrile or hydroxamic acid derivative thereof, said radical $R^3$ being attached to the thiazole ring by a carbon atom on the aliphatic chain.

According to GB—A—1,145,884 the compounds of formula A possess pharmacological activity particularly antiinflammatory activity. Examples of $R^1$ and $R^2$ are unsubstituted phenyl and phenyl substituted by halogen, lower alkyl, lower alkoxy, nitro, amino, substituted amino, mercapto, alkylthio, alkylsulphonyl or trihalomethyl.

One of the compounds of formula A has shown valuable antiinflammatory properties and has been made available for human administration under the generic name fentiazac. This compound is 4-(4-chlorophenyl)-2-phenylthiazole-5-acetic acid. The principal metabolite of fentiazac is disclosed by S. Pedrazzini in Boll. Chim. Farm., 1978, 117(1), 19—26 being 4-(4-chlorophenyl)-2-(4-hydroxyphenyl)thiazole-5-acetic acid. A solution of the metabolite in acetone is disclosed. No mention of pharmaceutical activity for the metabolite is made in this publication.

The anti-inflammatory activity of specific compounds of formula A against carrageenin-induced edema in the rat hind paw was extensively reported by Brown et al, in Journal of Medicinal Chemistry, 1974, Vol. 17 No. 11, pps. 1177 to 1181. Structure activity relationship revealed that the anti-inflammatory activity was found to be optimised when $R^2$ was chlorophenyl (i.e. fentiazac). However, the preferred $R^1$ group was found to be phenyl and 4-substitution of this ring reduced the anti-inflammatory activity. Thus 4-methoxy and 4-carboxy substitution both substantially reduced activity when $R^2$ was 4-chlorophenyl.

We have now surprisingly found a series of thiazole-5-acetic acids not specifically mentioned in the general formula (A) wherein $R^1$ represents a 4-substituted-phenyl, which possess marked anti-inflammatory activity when administered topically. Furthermore this series of thiazole-5-acetic acids possess low toxicity.

Accordingly this invention provides a semi-solid or aerosol pharmaceutical composition for topical administration comprising a compound having the formula I:

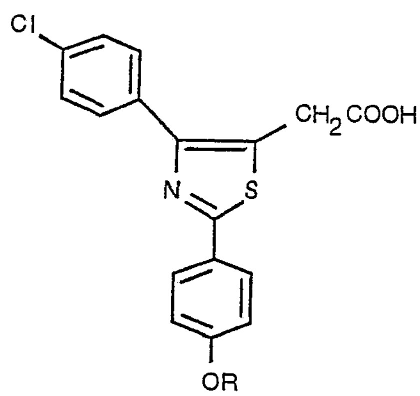

(I)

or a salt thereof, wherein R represents hydrogen or an alkanoyl group having 2 to 7 carbon atoms, preferably 2 to 5 carbon atoms, and a pharmaceutically acceptable topical carrier. Examples of R are acetyl, propionyl, butyryl, isobutyryl, valeryl and isovaleryl.

The compounds of formula I form salts, for example acid addition salts with acids such as hydrochloric and hydrobromic acid, or alkali metal (e.g. sodium or potassium) or alkaline earth metal (e.g. calcium) salts. Such salts may be prepared in known manner.

Compounds of formula I were tested for anti-inflammatory activity by the following procedure based on Tonelli et al, *Endocrinology 77*, 625 (1965):

Sprague-Dawley female rats, weighing 60 to 70 grams, are used in groups of 10. Ear edema is induced by inuncting both sides of the ear with an irritant mixture. This mixture containing 1% croton oil, 20% pyridine, 5% water and 74% diethyl ether, with or without test compound, is applied only once and only to the right ear. Six hours later the animals are sacrificed; a 9 mm diameter portion of both ears is punched out with a cork borer and weighed. The anti-inflammatory activity of the test agent is assessed by expressing the percent of the difference in average weight increase (Avg. inc.) between the ears of the control groups and of the treated group.

$$\% \text{ Inhibition} = 100 \times \frac{(\text{Avg. inc. in wt. of test groups}) - (\text{Avg. inc. in wt. of controls})}{(\text{Avg. inc. in wt. of controls})}$$

Results found for representative compounds of formula I are tabulated below, together with results found in 3 tests for the preferred compound reported by Brown et al in J.Med.Chem. (*loc-cit*) namely 4-(p-chlorophenyl)-2-phenyl-thiazole-5-acetic acid (B):

| Compound of formula I | Dose | % Inhibition | | |
|---|---|---|---|---|
| R = H | 50 μg | 16% | | |
| | 500 μg | 33% | | |
| | 2.5 mg | 86% | | |
| | 5 mg | 92% | | |
| R = $COCH_3$ | 50 μg | −14% | | |
| | 500 μg | 41% | | |
| | 2.5 mg | 85% | | |
| | 5 mg | 100% | | |
| R = $COC_4H_9$ | 50 μg | 41% | | |
| | 500 μg | 54% | | |
| | 2.5 mg | 71% | | |
| | 5 mg | 87% | | |
| 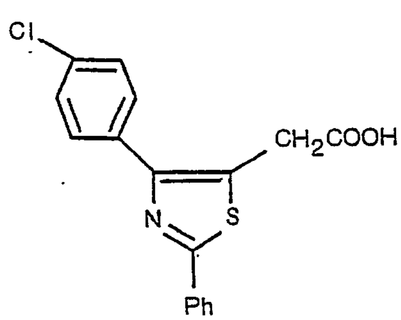 | 50 μg | −30, | 15, | −7% |
| | 500 μg | 10, | 35, | 52% |
| | 2.5 mg | 93, | 79, | 89% |
| | 5 mg | 92, | 93, | 93% |

(B) = Fentiazac

The compounds all show marked anti-inflammatory activity of about the same order.

Toxicity of compounds of formula I was measured by administering test compound orally to groups of 3 male and 3 female non-starved TFW mice at a series of dose levels. The results obtained for

the compound of formula I (R = H) and the preferred compound of formula B from J.Med.Chem. (loc-cit) are shown in the Table below:

| Dose Level mg/kg | No. Dead (time from dosing) Formula I (R = H) 24 hours | 7 days | Formula B 24 hours | 7 days |
|---|---|---|---|---|
| 450 | 0 | 0 | 2 | 3 |
| 675 | 1 | 1 | 3 | 6 |
| 1012.5 | 1 | 1 | 3 | 6 |
| 1518.8 | 1 | 1 | 2 | 4 |
| 2278.1 | 0 | 0 | 3 | 3 |
| 3417.2 | 0 | 0 | 5 | 6 |

These results show the compound 4-(p-chlorophenyl)-2-(p-hydroxyphenyl)thiazole-5-acetic acid to be considerably less toxic than the corresponding 2-phenyl analogue-fentiazac (Formula B).

This invention also provides compounds of formula Ia

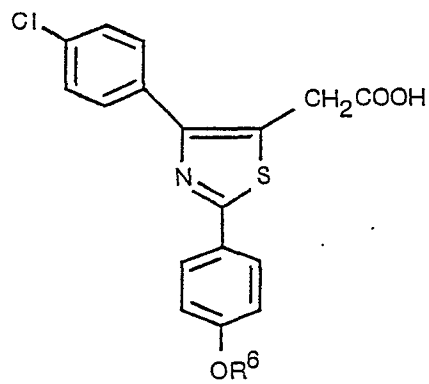

(Ia)

and salts thereof wherein $R^6$ represents an alkanoyl group having 2 to 7 carbon atoms and processes for preparing them. Such processes are outlined in GB—A—1,145,884 and GB—A—1,262,292. Accordingly this invention provides a process for preparing a compound of formula Ia or a salt thereof which comrpises —

(a) reacting an $\alpha$-haloketone of general formula II:

p-chlorophenyl-CO—CH($hal$)$CH_2COOH$ (II)

wherein hal is a halogen atom, with a thioamide of general formula III:

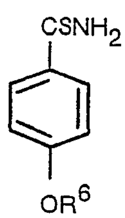

(III)

wherein R is as hereinbefore defined, or

(b) dehydrating a compound of formula

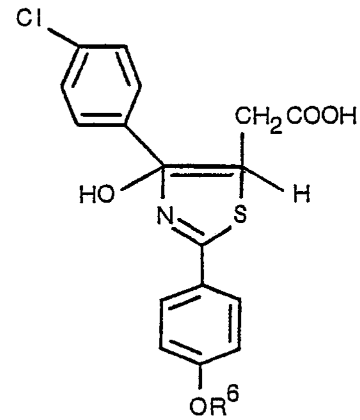

(IV)

wherein $R^6$ is as hereinbefore defined to give a corresponding compound of formula I, or

(c) acylating a compound of formula I wherein R is hydrogen to give a compound of formula Ia as defined hereinabove, or

(d) a compound of formula Ia is converted to a salt thereof or a salt is converted to a compound of formula Ia.

The compounds of formula I wherein R is H may be prepared by processes analogous to processes a) and b) above and also by dealkylating a compound of formula V

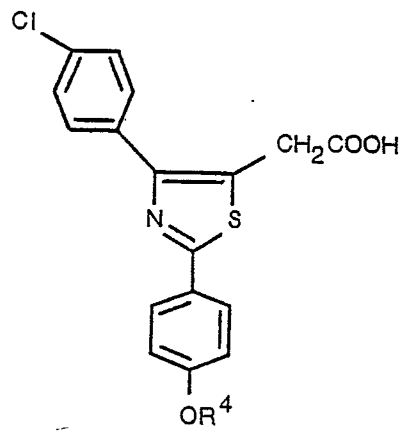

(V)

wherein $R^4$ is alkyl or aralkyl.

Methods for carrying out processes (a) and (b) above are extensively described in our GB—A—1,145,884 and 1,262,292.

Acylation of a compound of formula I wherein R is hydrogen may be effected by standard methods using an acylating agent comprising an acyl R moiety, such as an anhydride or acyl halide e.g. chloride.

Methods for carrying out dealkylation of a compound of formula V are well known in the art, for example treating the ethers with hydrogen bromide, hydrogen iodide or boron tribromide. Preferably $R^4$ is alkyl of 1 to 4 carbon atoms, most preferably methyl.

By a 'semi-solid pharmaceutical composition' is meant an ointment, cream, salve, paste, jelly or other pharmaceutically or cosmetic composition of substantially similar consistency suitable for application to the skin. Examples of semi-solid compositions are given in Chapter 17 of The Theory and Practice of Industrial Pharmacy, Lachman, Lieberman and Kanig, published by Lea and Febiger (1970) and in Chapter 67 of Remington's Pharmaceutical Sciences, 15th Edition (1975) published by Mack Publishing Company.

Preferably, the topical compositions of the present invention contain from 0.1% to 20% by weight of the active ingredient. The compositions may, for example, contain from 0.5% (preferably from 1%) to 10% by weight of the active ingredient.

The carrier used in the topical compositions of the present invention may be any carrier suitable

for preparing topical semi-solid compositions or topical aerosol compositions. Examples of suitable carriers for semi-solid compositions are given in Lachman, Lieberman and Kanig (*loc-cit*) and in Chapter 67 of Remington's Pharmaceutical Sciences, (*loc-cit*). The carrier for the semi-solid composition may be, for example an emulsion base of the oil in water class (e.g. an emulsion of soft and liquid paraffins in water). Alternatively, the carrier may be an absorption base (e.g. a mixture of wool fat and soft paraffin). A third class of suitable carriers are water miscible bases (e.g. mixtures of high and low molecular weight polythene glycols).

When the composition is in aerosol form for topical administration, the composition may comprise the active ingredient and an easily liquifiable gas. Examples of such liquifiable gases are halogenated hydrocarbons and liquified lower hydrocarbons, both of which are well known as propellants in the aerosol art. (By "lower hydrocarbon" is meant a hydrocarbon containing up to six carbon atoms).

In addition to the active ingredient and the carrier base, the compositions of the invention may contain other ingredients such as antioxidants, buffers, emulsifying agents, perfumes, preservatives and solvents which confer on the product properties desirable in a topical formulation. In particular, buffers may be employed to adjust the pH of the composition to within the range of, for example 4 to 5.5 (e.g. 4.8) to maintain the active ingredient in its free acid form. The compositions can also contain other active ingredients.

In a further aspect, the invention provides the compounds for use in treating inflammation in warm blooded animals. The treatment is affected by topically administering to the animal an anti-inflammatory effective amount of a compound of formula I. By "topically administering" is meant administering to the exterior skin surface. The active ingredient may be administered in the form of a composition of the present invention.

Example 1

4-(4-Chlorophenyl)-2-(4-hydroxyphenyl)thiazole-5-acetic acid

4-(4-Chlorophenyl)-2-(4-methoxyphenyl)thiazole-5-acetic acid (1.26 g, 3.5 mmol) was heated to reflux in a mixture of glacial acetic acid (10 $cm^3$) and 48% hydrobromic acid (20 $cm^3$) for 4 hours. On cooling, the hydrobromide salt of the title compound crystallised (1.03 g). The crystals were collected, washed with water and ether, and dried m.p. 239—241° (decomp).

Analysis:

Found: C 47.7; H 3.1; N 2.4; ionic bromide 19.2.

$C_{17}H_{12}ClNO_3S{\cdot}HBR$ requires C 47.8; H 3.1; N 3.3; ionic bromine 18.7%.

Example 2

4-(4-Chlorophenyl)-2-(4-hydroxyphenyl)thiazole-5-acetic acid

3-Bromo-3-(4-chlorobenzoyl)propionic acid (27 g), and 4-hydroxy-thiobenzamide (14.6 g) were heated to 80° in dimethylformamide (50 ml). The reactants were kept at this temperature for 1 hour, cooled and poured onto ice. The resulting gum solidified, and was filtered, and washed with water, to give 31.6 g of powder, m.p. 184—194°C (decomp.). This was recrystallised from aqueous isopropanol affording 25.4 g of the title compound, hemihydrate, m.p. 192—194°(d).

Analysis:

Found: C 57.7; H 3.5; N 3.6.

$C_{17}H_{12}ClNO_3S{\cdot}\frac{1}{2}H_2O$ requires: C 57.55; H 3.7; N 3.9%.

Example 3

2-(4-Acetoxyphenyl)-4-(4-chlorophenyl)thiazole-5-acetic acid

4-(4-Chlorophenyl)-2-(4-hydroxyphenyl)-thiazole-5-acetic acid (7.0 g, 0.016 moles) was dissolved in 0.1 N sodium hydroxide (493 ml, 0.372 moles) and cooled to 0°C. Acetic anhydride (1.5 ml, 0.016 moles) was added and the mixture left standing at room temperature for 3 hours. To the solution was added dilute hydrochloric acid and the resulting precipitate was filtered off, washed with a little water, dried and recrystallised from methylethylketone to give the title compound as a colourless solid (2.4 gm), m.p. 177—180°C.

Analysis:

Found: C 58.94; H 3.87; N 3.43%.

$C_{19}H_{14}ClNO_4S$ requires: C 58.84; H 3.64; N 3.61%.

Example 4

4-(4-Chlorophenyl)-2-(4-Valeryloxyphenyl)thiazole-5-acetic acid

4-[4-Chlorophenyl]-2-[4-hydroxyphenyl]-thiazole-5-acetic acid (3.81 g, 0.011 moles) was dissolved in 0.2 N sodium hydroxide (55 ml, 0.027 moles) and cooled to 0°C. Valeryl anhydride (2.0 g, 0.011 moles) was added and the reaction flask shaken vigorously for 4 minutes. Dilute hydrochloric was added, and the resulting gum extracted into chloroform. The chloroform layer was washed with water, separated, dried over magnesium sulphate to give a white solid. The solid was stirred with water at about 50°C for 1/4 hour, filtered and dried to give the title compound as a colourless solid (3.68 g), m.p. 197—9°C.

Analysis:
Found: C 61.16; H 4.61; N 3.11;
$C_{22}H_{20}ClNO_4S$ requires: C 61.46; H 4.69; N 3.26.

Example 5

4-(4-Chlorophenyl)-2-(4-hydroxyphenyl)thiazole-5-acetic acid

By a process analogous to Example 2, methyl-3-bromo-3-(4-chlorobenzoyl)propionate and 4-hydroxy-thiobenzamide are reacted to give methyl 4-(4-chlorophenyl)-2-(4-hydroxyphenyl)thiazole-5-acetate. This compound is hydrolysed using 2N sodium hydroxide to give the title compound.

Example 6

4-(4-Chlorophenyl)-2-(4-hydroxyphenyl)thiazole-5-acetic acid

2-(4-Acetoxyphenyl)-4-(4-chlorophenyl)-thiazole-5-acetic acid (prepared according to Example 3) is hydrolysed using 2N sodium hydroxide to give the title compound.

Example 7

4-(4-Chlorophenyl)-2-(4-hydroxyphenyl)thiazole-5-acetic acid

3-Bromo-3-(4-chlorobenzoyl)propionic acid and an equimolar amount of 4-hydroxythiobenzamide are stirred in isopropyl alcohol solvent containing sodium carbonate to give 4-(4-chlorophenyl)-4-hydroxy-2-(4-hydroxyphenyl)-2-thiazolin-5-acetic acid. This compound is dehydrated by heating to give the title compound.

**Claims for the Contracting States DE NL SE**

1. A semi-solid or aerosol pharmaceutical composition for topical administration comprising a compound of formula I

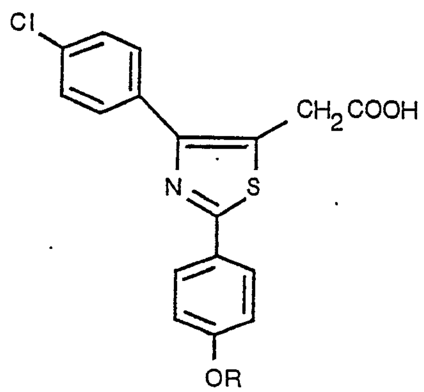

(I)

or a pharmaceutically acceptable salt thereof, wherein R represents hydrogen or an acyl group derived from a carboxylic acid, and a pharmaceutically acceptable topical carrier.

2. A composition as claimed in Claim 1 in which the compound of formula I is 4-(4-chlorophenyl)-2-(4-hydroxyphenyl)thiazole-5-acetic acid or a pharmaceutically acceptable salt thereof.

3. A composition as claimed in Claim 1 in which the compound of formula I is 2-(4-acetoxyphenyl)-4-(4-chlorophenyl)thiazole-5-acetic acid or a pharmaceutically acceptable salt thereof.

4. A composition as claimed in Claim 1 in which the compound of formula I is 4-(4-chlorophenyl)-2-(4-valeryloxyphenyl)thiazole-5-acetic acid or a pharmaceutically acceptable salt thereof.

5. A compound of formula Ia

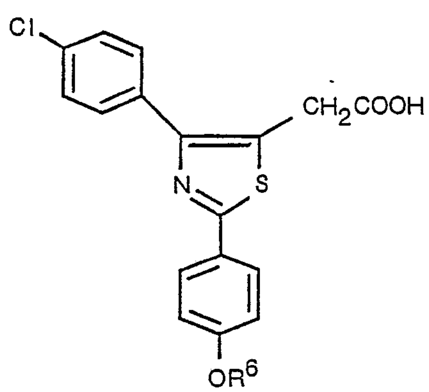

(Ia)

or a salt thereof, wherein $R^6$ represents an alkanoyl group having 2 to 7 carbon atoms.

6. A compound of formula Ia as claimed in Claim 5 which is 2-(4-acetoxyphenyl)-4-(4-chlorophenyl)-thiazole-5-acetic acid or 4-(4-chlorophenyl)-2-(4-valeryloxyphenyl)thiazole-5-acetic acid, or a salt thereof.

7. A compound of formula Ia as defined in Claim 5 for use as a topical anti-inflammatory agent.

8. A process for preparing a compound as claimed in Claim 5 characterised in that:

(a) an $\alpha$-haloketone of general formula II:

p-chlorophenyl-CO—CHCH$_2$COOH (II)
                                  |
                                 hal

wherein hal is a halogen atom, is reacted with a thioamide of general formula III:

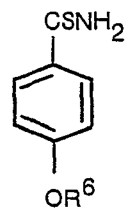

(III)

wherein $R^6$ is as hereinbefore defined, or

(b) a compound of formula IV

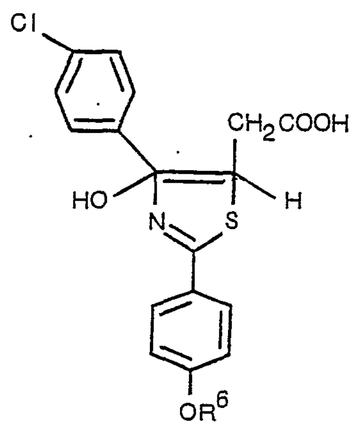

(IV)

wherein $R^6$ is as hereinbefore defined is dehydrated to give a corresponding compound of formula Ia or

(c) a compound of formula I as defined in Claim 1 wherein R is hydrogen is acylated to give a compound of formula Ia as defined hereinbefore or

(d) a compound of formula Ia is converted to a salt thereof or a salt is converted to a compound of formula Ia.

**Claims for the Contracting State AT**

1. A process for preparing a semi-solid or aerosol pharmaceutical composition for topical administration characterised in that a compound of formula I

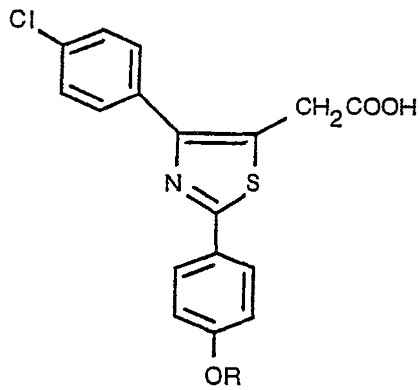

(I)

or a pharmaceutically acceptable salt thereof wherein R represents hydrogen or an alkanoyl group having 2 to 7 carbon atoms and a pharmaceutically acceptable topical carrier are brought into a form suitable for therapeutic administration.

2. A process as claimed in Claim 1 in which the compound of formula I is 4-(4-chlorophenyl)-2-(4-hydroxyphenyl)thiazole-5-acetic acid or a pharmaceutically acceptable salt thereof.

3. A process as claimed in Claim 1 in which the compound of formula I is 2-(4-acetoxyphenyl)-4-(4-chlorophenyl)-thiazole-5-acetic acid or a pharmaceutically acceptable salt thereof.

4. A process as claimed in Claim 1 in which the compound of formula I is 4-(4-chlorophenyl)-2-(4-valeryloxyphenyl)thiazole-5-acetic acid or a pharmaceutically acceptable salt thereof.

5. A process for preparing a compound of formula Ia

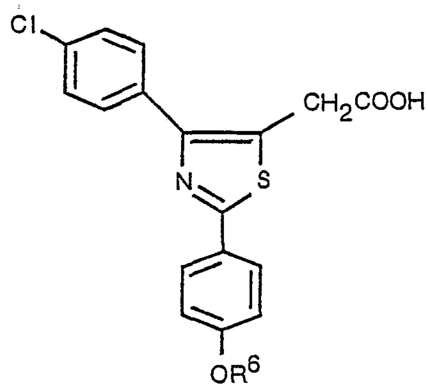

(Ia)

or a salt thereof, wherein $R^6$ represents an alkanoyl group having 2 to 7 carbon atoms, characterised in that:

(a) an $\alpha$-haloketone of general formula II:

p-chlorophenyl-CO—$CHCH_2COOH$ (II)
|
hal

wherein hal is a halogen atom, is reacted with a thioamide of general formula III:

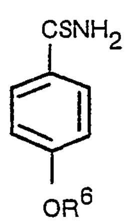

(III)

wherein $R^6$ is as hereinbefore defined, or

(b) a compound of formula IV

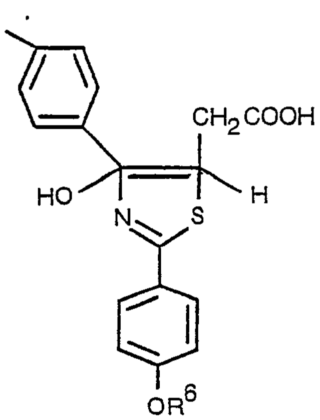

(IV)

wherein $R^6$ is as hereinbefore 20 defined is dehydrated to give a corresponding compound of formula Ia, or

(c) a compound of formula I wherein R is hydrogen is acylated to give a compound of formula Ia, as defined hereinabove, or

(d) a compound of formula Ia is converted to a salt thereof or a salt is converted to a compound of formula Ia.

6. A process as claimed in Claim 5, in which the compound prepared is 2-(4-acetoxyphenyl)-4-(4-chlorophenyl)-thiazole-5-acetic acid.

7. A process as claimed in Claim 5, in which the compound is 4-(4-chlorophenyl)-2-(4-valeryloxyphenyl)-thiazole-5-acetic acid, or a salt thereof.

8. A process as claimed in Claim 1 in which the compound of formula I or salt thereof is prepared by a process as claimed in any one of Claims 5 to 7.

**Patentansprüche für die Vertragstaaten DE NL SE:**

1. Pharmazeutische halbfeste oder Aerosol-Zusammensetzung zur topischen Verabreichung, die eine Verbindung der Formel (I)

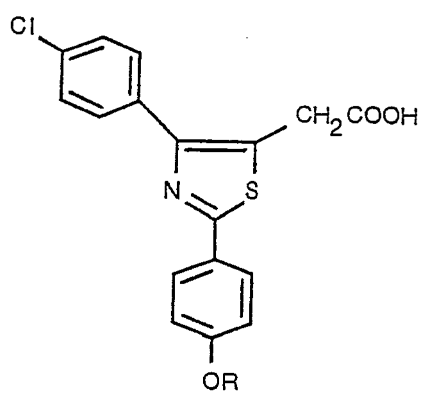

(I)

oder ein pharmazeutisch annehmbares Salz hievon, wobei R Wasserstoff oder eine von einer Carbonsäure stammende Acylgruppe bedeutet, und einen pharmazeutisch annehmbaren topischen Träger aufweist.

2. Zusammensetzung wie in Anspruch 1 beansprucht, worin die Verbindung der Formel (I) 4-(4-Chlorphenyl)-2-(4-hydroxyphenyl)-thiazol-5-essigsäure oder ein pharmazeutisch annehmbares Salz hievon ist.

3. Zusammensetzung wie in Anspruch 1 beansprucht, worin die Verbindung der Formel (I) 2-(4-Acetoxyphenyl)-4-(4-chlorphenyl)-thiazol-5-essigsäure oder ein pharmazeutisch annehmbares Salz hievon ist.

4. Zusammensetzung wie in Anspruch 1 beansprucht, worin die Verbindung der Formel (I) 4-(4-Chlorphenyl)-2-(4-valeryloxyphenyl)-thiazol-5-essigsäure oder ein pharmazeutisch annehmbares Salz hievon ist.

5. Eine Verbindung der Formel (Ia)

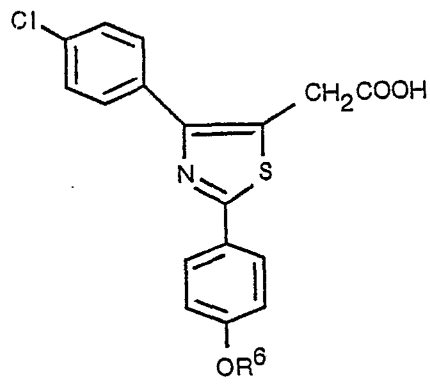

(Ia)

oder ein Salz hievon, worin $R^6$ eine Alkanoylgruppe mit 2 bis 7 C-Atomen darstellt.

6. Eine Verbindung der Formel (Ia) wie in Anspruch 5 beansprucht, die 2-(4-Acetoxyphenyl)-4-(4-chlorphenyl)-thiazol-5-essigsäure oder 4-(4-Chlorphenyl)-2-(4-valeryloxyphenyl)-thiazol-5-essigsäure oder ein Salz hievon ist.

7. Eine Verbindung der Formel (Ia) wie in Anspruch 5 definiert, zur Verwendung als topisches entzündungshemmendes Mittel.

8. Verfahren zum Herstellen einer Verbindung wie in Anspruch 5 beansprucht, dadurch gekennzeichnet, daß

(a) ein $\alpha$-Halogenketon der allgemeinen Formel (II)

p-Chlorphenyl-CO—$CHCH_2COOH$ (II)
|
Hal

worin Hal ein Halogenatom bedeutet, mit einem Thioamid der allgemeinen Formel (III)

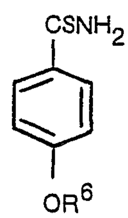

(III)

worin $R^6$ wie oben definiert ist, umgesetzt wird oder

(b) eine Verbindung der Formel (IV)

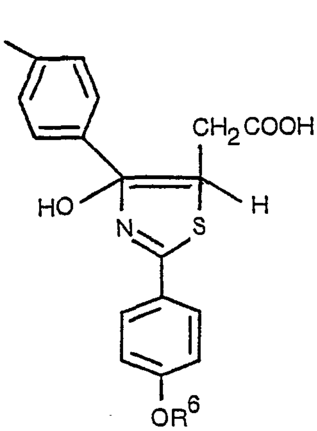

(IV)

worin $R^6$ wie oben definiert ist, zu einer entsprechenden Verbindung der Formel (Ia) dehydratisiert wird, oder

(c) eine Verbindung der Formel (I) wie in Anspruch 1 definiert, worin R Wasserstoff ist, zu einer Verbindung der Formel (Ia), wie oben definiert, acyliert wird, oder

(d) eine Verbindung der Formel (Ia) in ein Salz hievon oder ein Salz in eine Verbindung der Formel (Ia) überführt wird.

**Patentansprüche für den Vertragsstaat: AT**

1. Verfahren zum Herstellen einer pharmazeutischen halbfesten oder Aerosol-Zusammensetzung zur topischen Verabreichung, dadurch gekennzeichnet, daß eine Verbindung der Formel (I)

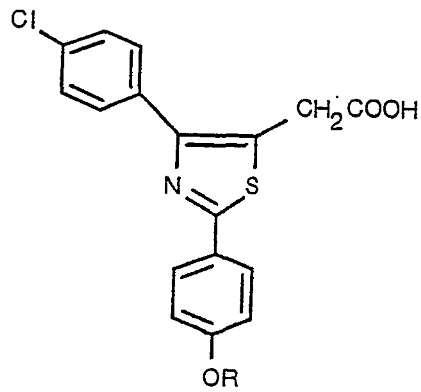

(I)

oder ein pharmazeutisch annehmbares Salz hievon, worin R Wasserstoff oder eine Alkanoylgruppe mit 2 bis 7 C—Atomen bedeutet, und ein pharmazeutisch annehmbarer topischer Träger in eine für therapeutische Verabreichung geeignete Form gebracht werden.

2. Verfahren wie in Anspruch 1 beansprucht, wobei die Verbindung der Formel (I) 4-(4-Chlorphenyl)-2-(4-hydroxyphenyl)-thiazol-5-essigsäure oder ein pharmazeutisch annehmbares Salz hievon ist.

3. Verfahren wie in Anspruch 1 beansprucht, wobei die Verbindung der Formel (I) 2-(4-Acetoxyphenyl)-4-(4-chlorphenyl)-thiazol-5-essigsäure oder ein pharmazeutisch annehmbares Salz hievon ist.

4. Verfahren wie in Anspruch 1 beansprucht, worin die Verbindung der Formel (I) 4-(4-Chlorphenyl)-2-(4-valeryloxyphenyl)-thiazol-5-essigsäure oder ein pharmazeutisch annehmbares Salz hievon ist.

5. Verfahren zur Herstellung einer Verbindung der Formel (Ia)

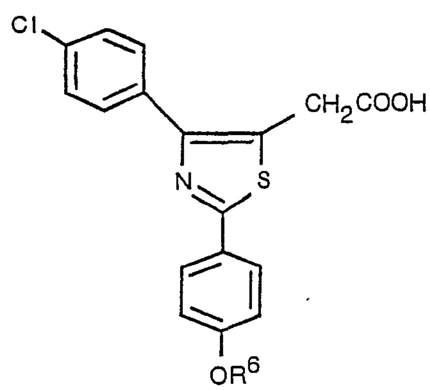

(Ia)

oder eines Salzes hievon, worin $R^6$ eine Alkanolygruppe mit 2 bis 7 C—Atomen darstellt, dadurch gekennzeichnet, daß

(a) ein $\alpha$-Halogenketon der allgemeinen Formel (II)

p-Chlorphenyl-CO—$CHCH_2COOH$ (II)
Hal

worin Hal ein Halogenatom bedeutet, mit einem Thioamid der allgemeinen Formel (III)

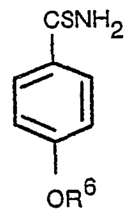

(III)

worin $R^6$ wie oben definiert is, umgesetzt wird, oder (b) eine Verbindung der Formel (IV)

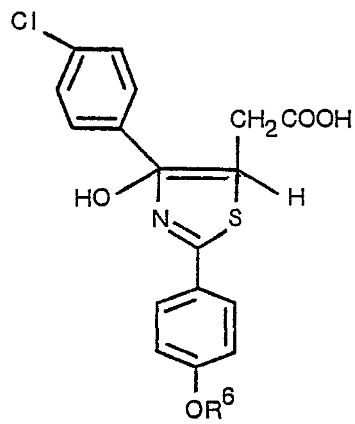

(IV)

worin $R^6$ wie oben definiert ist, zu einer entsprechenden Verbindung der Formel (Ia) dehydratisiert wird, oder

(c) eine Verbindung der Formel (I), worin R Wasserstoff ist, zu einer Verbindung der Formel (Ia), wie oben definiert, acyliert wird oder

(d) eine Verbindung der Formel (Ia) in ein Salz hievon oder ein Salz in eine Verbindung der Formel (Ia) überführt wird.

6. Verfahren wie in Anspruch 5 beansprucht, worin die hergestellte Verbindung 2-(4-Acetoxyphenyl)-4-(4-chlorphenyl)-thiazol-5-essigsäure ist.

7. Verfahren wie in Anspruch 5 beansprucht, worin die hergestellte Verbindung 4-(4-Chlorphenyl)-2-(4-valeryloxyphenyl)-thiazol-5-essigsäure oder ein Salz hievon ist.

8. Verfahren wie in Anspruch 1 beansprucht, worin die Verbindung der Formel (I) oder ein Salz hievon nach einem Verfahren, wie in einem der Ansprüche 5 bis 7 beansprucht, hergestellt ist.

**Revendications pour les états Contractants: DE NL SE**

1. Composition pharmaceutique semi-solide ou en aérosol pour l'administration topique, comprenant un composé de formule I

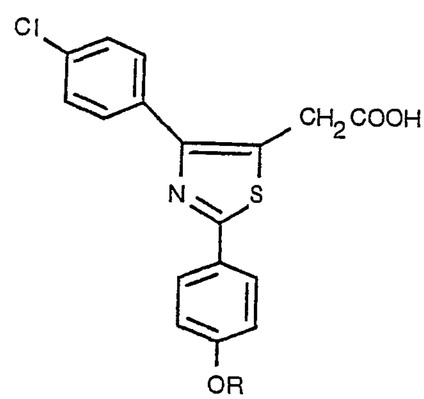

(I)

ou un sel pharmaceutiquement acceptable de ce composé, formule dans laquelle R représente l'hydrogène ou un groupe acyle dérivé d'un acide carboxylique, et un support topique pharmaceutiquement acceptable.

2. Composition suivant la revendication 1, dans laquelle le composé de formule I est l'acide 4-(4-chlorophényl)-2-(4-hydroxyphényl)thiazole-5-acétique ou un sel pharmaceutiquement acceptable de cet acide.

3. Composition suivant la revendication 1, dans laquelle le composé de formule I est l'acide 2-(4-acétoxyphényl)-4-(4-chlorophényl)thiazole-5-acétique ou un sel pharmaceutiquement acceptable de cet acide.

4. Composition suivant la revendication 1, dans laquelle le composé de formule I est l'acide 4-(4-chlorophényl)-2-(4-valéryloxyphényl)thiazole-5-acétique ou un sel pharmaceutiquement acceptable de ce composé.

5. Composé de formule Ia

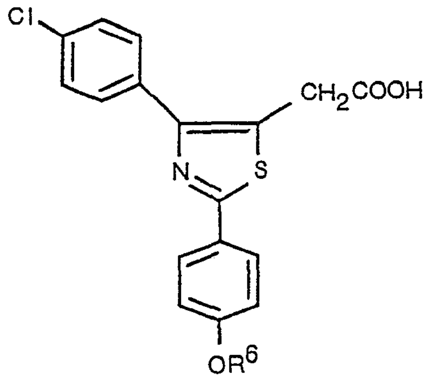

(Ia)

ou sel de ce composé, formule dans laquelle $R^6$ représente un groupe alcanoyle ayant 2 à 7 atomes de carbone.

6. Composé de formule Ia suivant la revendication 5, qui est l'acide 2-(4-acétoxyphényl)-4-(4-chlorophényl)thiazole-5-acétique ou l'acide 4-(4-chlorophényl)-2-(4-valéryloxyphényl)thiazole-5-acétique ou un sel de ces acides.

7. Composé de formule Ia suivant la revendication 5 destiné à être utilisé comme agent anti-inflammatoire topique.

8. Procédé de préparation d'un composé suivant la revendication 5, caractérisé en ce que:

(a) on fait réagir une $\alpha$-halogénocétone de formule générale II:

$$\text{p-chlorophényl-CO—}\underset{\text{hal}}{\underset{|}{\text{C}}}\text{HCH}_2\text{COOH} \qquad \text{(II)}$$

dans laquelle hal est un atome d'halogène, avec un thioamide de formule générale III:

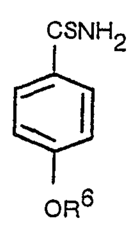

(III)

dans laquelle $R^6$ a la définition donnée ci-dessus, ou

(b) on déshydrate un composé de formule IV

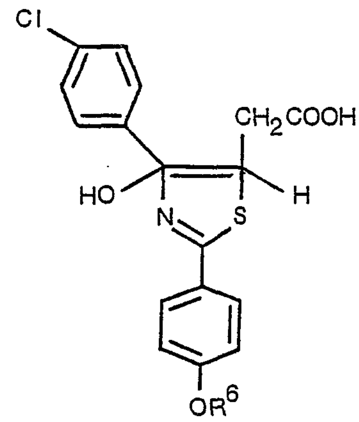

(IV)

dans laquelle $R^6$ a la définition donnée ci-dessus, pour obtenir un composé correspondant de formule Ia, ou

(c) on acyle un composé de formule I comme défini dans la revendication 1, dans laquelle R est l'hydrogène pour obtenir un composé de formule Ia comme défini ci-dessus, ou

(d) on convertit un composé de formule Ia en un sel de ce composé ou on convertit un sel en un composé de formule Ia.

**Revendications pour l'etat Contractant: AT**

1. Procédé de préparation d'une composition pharmaceutique semi-solide ou en aérosol pour l'administration topique, caractérisé en ce qu'on fait prendre une forme qui convient à l'administration thérapeutique à un composé de formule I

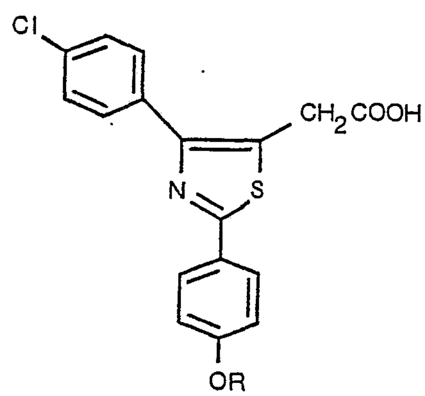

(I)

ou à un sel pharmaceutiquement acceptable de ce composé, formule dans laquelle R représente l'hydrogène ou un groupe alcanoyle ayant 2 à 7 atomes de carbone, et à un support topique pharmaceutiquement acceptable.

2. Procédé suivant la revendication 1, dans lequel le composé de formule I est l'acide 4-(4-chlorophényl)-2-(4-hydroxyphényl)thiazole-5-acétique ou un sel pharmaceutiquement acceptable de ce acide.

3. Procédé suivant la revendication 1, dans lequel le composé de formule I est l'acide 2-(4-acétoxyphényl)-4-(4-chlorophényl)thiazole-5-acetique ou un sel pharmaceutiquement acceptable de cet acide.

4. Procédé suivant la revendication 1, dans lequel le composé de formule I est l'acide 4-(4-chlorophényl)-2-(4-valéryloxyphényl)thiazole-5-acétique ou un sel pharmaceutiquement acceptable de cet acid.

5. Procédé de préparation d'un composé de formule Ia

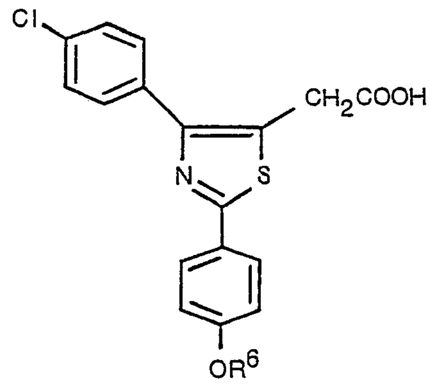

(Ia)

ou d'un sel de ce composé, formule dans laquelle $R^6$ représente un groupe alcanoyle ayant 2 à 7 atomes de carbone, caractérisé en ce que:

(a) on fait réagir une $\alpha$-halogénocétone de formule générale II:

p-chlorophényl-CO—CH$CH_2$COOH (II)
|
hal

dans laquelle hal désigne un atome d'halogène, avec un thio-amide de formule générale III:

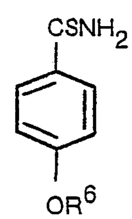

(III)

dans laquelle $R^6$ a la définition donnée ci-dessus, ou

(b) on déshydrate un composé de formule IV

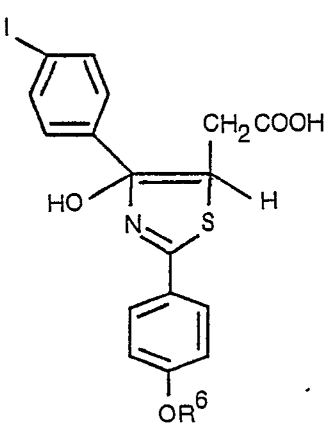

(IV)

dans laquelle $R^6$ a la définition donnée ci-dessus, pour obtenir un composé correspondant de formule Ia, ou

(c) on acyle un composé de formule I dans laquelle R est l'hydrogène, pour obtenir un composé de formule Ia comme défini ci-dessus, ou

(d) on convertit un composé de formule Ia en un sel de ce composé ou on convertit un sel en un composé de formule Ia.

6. Procédé suivant la revendication 5, dans lequel le composé préparé est l'acide 2-(4-acétoxyphényl)-4-(4-chlorophényl)thiazole-5-acétique.

7. Procédé suivant la revendication 5, dans lequel le composé est l'acide 4-(4-chlorophényl)-2-(4-valéryloxyphényl)thiazole-5-acétique ou un sel de cet acide.

8. Procédé suivant la revendication 1, dans lequel le composé de formule I ou un sel de ce composé est préparé par un procédé suivant l'une quelconque des revendications 5 à 7.